Europäisches Patentamt

European Patent Office

Office européen des brevets

(19)

(11) Publication number: **0 201 882**
A2

(12) **EUROPEAN PATENT APPLICATION**

(21) Application number: 86106359.2

(22) Date of filing: 09.05.86

(51) Int. Cl.⁴: **C 12 N 15/00**
C 07 K 13/00, C 12 N 1/20
C 12 P 21/02
//(C12N1/20, C12R1:19)

(30) Priority: 10.05.85 JP 99094/85
12.10.85 JP 227531/85
01.04.86 JP 75210/86

(43) Date of publication of application:
20.11.86 Bulletin 86/47

(84) Designated Contracting States:
DE FR GB

(71) Applicant: KYOWA HAKKO KOGYO CO., LTD.
6-1, Ohte-Machi Itchome
Chiyoda-ku Tokyo(JP)

(72) Inventor: Kuwana, Yoshihisa
3-6-6, Asahi-machi
Machida-shi Tokyo(JP)

(72) Inventor: Kuga, Tetsuro
3-9-9, Naka-machi
Machida-shi Tokyo(JP)

(72) Inventor: Sato, Moriyuki
2730-15, Naruse
Machida-shi Tokyo(JP)

(72) Inventor: Itoh, Seiga
218-14, Aza Hachimannishi Aihara
Sagamihara-shi Kanagawa-ken(JP)

(74) Representative: VOSSIUS & PARTNER
Siebertstrasse 4 P.O. Box 86 07 67
D-8000 München 86(DE)

(54) Fish prolactin polypeptide and derivatives thereof.

(57) According to the present invention, a recombinant DNA wherein a DNA coding for a fish prolactin polypeptide or a derivative thereof is incorporated and a microorganism containing the recombinant DNA are obtained and they can be used for mass production of the fish prolactin polypeptide and the derivative thereof.

EP 0 201 882 A2

# FISH PROLACTIN POLYPEPTIDE AND DERIVATIVES THEREOF

The present invention relates to a DNA coding for a fish prolactin polypeptide, for example, the prolactin polypeptide of Oncorhynchus keta, or a derivative thereof, a recombinant DNA wherein said DNA is incorporated, a microorganism containing the recombinant DNA and a process for the production of the fish prolactin polypeptide or a derivative thereof using the microorganism. The fish prolactin and its derivatives have the effect of stimulating the growth of fish.

It is known that adult Oncorhynchus keta has adaptability to fresh water from the fact that when it is put in fresh water osmotic pressure of its plasma slightly decreases after 24 hours and is kept at a constant level thereafter. Prolactin seems to have an important role in adaptation of Oncorhynchus keta to fresh water because the concentration of prolactin in blood increases significantly after 24 hours coincidentally with the decrease of osmotic pressure.

Mammalian prolactins are produced in the pituitary gland. The activity and structure of the mammalian prolactins are known. For example, human prolactin has been reported in Endocrinology 71, 209 (1962) by J. B. Josimovich, et al., and in Proc. Natl. Acad. Sci. USA 58, 2307 (1967) by L. M. Sherwood, et al.

As for fish prolactin, there have been some reports on the isolation thereof, examples of which are given below.

Isolation from tilapias
    S. W. Farmer, et al., Gen. Comp. Endocrin., 31, 60-71 (1977)

Isolation from salmons
    D. R. Idler, et al., Gen. Comp. Endocrin., 35, 409-418 (1978)

Isolation from carp T.B. Ng, et al., Gen. Comp. Endocrin., 42, 141-146 (1980)

Isolation from teleosts H. Kawauchi, et al., Gen. Comp. Endocrin., 49, 446-458 (1983)

On the other hand, as for mammalian prolactin genes, rat prolactin gene [N. E. Cooke, et al., J. Biol. Chem. 255(13), 6502 (1980)], bovine prolactin gene [J. H. Nilson, et al., Nucleic Acids Res., 8(7), 1561 (1980)] and human prolactin gene [N. E. Cooke, et al., J. Biol. Chem., 256(8), 4007 (1981)] are already known. A fish prolactin gene has not been reported yet.

Fish prolactins have the effect of stimulating the growth of fish and the effect of adjusting the adaptability of fish to fresh water, and thus are useful as a component of baits for fish cultivation. The amount of the prolactin provided by the recovery from the fish pituitary gland is limited. Therefore, it has been desired that a process for providing a large amount of fish prolactin at a low cost is developed.

The present inventors studied a method of producing fish prolactins by recombinant DNA techniques. As the result, the present inventors have successfully recovered a DNA complementary to a fish prolactin polypeptide and a derivative thereof which are usable in the production of the fish prolactin polypeptide and a derivative thereof, and produced a recombinant DNA and a microorganism containing the DNA. That is, a messenger RNA (mRNA) was extracted from the pituitary gland of Oncorhynchus keta and a DNA (cDNA) complementary to the mRNA was synthesized. Then, a DNA probe corresponding to the amino acid sequence around the N-terminal of the prolactin

of Oncorhynchus keta was synthesized and a gene of the prolactin of Oncorhynchus keta was cloned by selecting a cDNA which hybridized with the DNA probe. Then, the whole base sequence of the cDNA of the gene was determined.

The present inventors have further studied and found that a large amount of prolactin of Oncorhynchus keta is formed and accumulated in a medium by culturing a microorganism containing a recombinant DNA wherein a DNA coding for the prolactin of Oncorhynchus keta is incorporated. Further, the present inventors have studied and found that a large amount of a prolactin polypeptide derivative of Oncorhynchus keta is formed and accumulated in a medium by culturing a microorganism containing a recombinant DNA wherein a DNA coding for the prolactin polypeptide derivative of Oncorhynchus keta is incorporated.

Fig. 1 (1) and (2) are flow sheets for synthesizing cDNA by the method of Okayama-Berg and constructing a recombinant plasmid containing the DNA.

Fig. 2 illustrates the restriction enzyme map of the cDNA in pSPRL1.

Fig. 3 is a flow sheet for constructing the recombinant plasmids pSPRLA6 and pSPRLB1.

Fig. 4 is a flow sheet for constructing plasmids pSPRLQ28 and pSPRLQ52.

Fig. 5 is a flow sheet for constructing plasmid pTrS20.

The present invention provides a fish prolactin polypeptide, for example, the polypeptide having the peptide sequence as illustrated in Table 1 and a derivative thereof. The polypeptide can be produced by recombinant DNA techniques in the following manner.

That is, an mRNA of fish prolactin is used as a template to prepare a DNA (cDNA) complementary to the mRNA, and then a recombinant plasmid wherein the cDNA or a derivative thereof is incorporated is prepared. Further, the recombinant plasmid is incorporated in a host microorganism. The DNA and recombinant plasmid can be used for amplification of a prolactin gene or a derivative thereof in bacteria such as Escherichia coli. Microorganisms carrying the recombinant plasmid are useful for producing a large amount of fish prolactin polypeptide and derivatives thereof at low cost.

Thus, the present invention provides a DNA coding for a fish prolactin polypeptide or a derivative thereof, a recombinant DNA wherein the DNA is incorporated, a microorganism containing the recombinant DNA and a process for producing the fish prolactin polypeptide and the derivative using the microorganism.

The DNA and recombinant plasmid of the present invention are prepared by the following general method.

Whole RNA is prepared from the pituitary gland of Oncorhynchus keta and passed through an oligo(dT) cellulose column to isolate RNA having polyadenylic acid [poly(A)] [poly(A)RNA]. A double stranded DNA is synthesized using the poly(A)RNA as a template and a reverse transcriptase. A recombinant is obtained using in vitro recombinant DNA techniques by inserting the synthetic DNA into a vector DNA such as Escherichia coli plasmid DNA. A recombinant plasmid having the DNA complementary to the prolactin mRNA of Oncorhynchus keta is selected based on the marker carried on the plasmid.

A DNA coding for a fish prolactin polypeptide derivative is obtained by treating the DNA coding for the fish prolactin polypeptide with an enzyme. The DNA is inserted into a vector DNA to obtain a recombinant DNA and the recombinant DNA is incorporated into a microorganism to obtain a transformant. A fish prolactin polypeptide derivative is obtained by culturing the transformant.

A process for producing the DNA and recombinant plasmid of the present invention is explained in detail below.

The pituitary gland is excised from captured Oncorhynchus keta and immediately freezed in a liquid nitrogen. Guanidium isothiocyanate is added to the freezed pituitary gland and the pituitary gland is disrupted and solubilized. Then, the solubilized pituitary gland is put on CsCl solution layer and subjected to ultra centrifugation to obtain whole cytoplasmic RNA as a precipitate. Alternatively, LiCl is added to the solubilized matter with guanidium isothiocyanate to recover only RNA as a precipitate.

The extracted RNA is dissolved in an NaCl or KCl hypertonic solution (for example 0.5M) and passed through an oligo(dT) cellulose clumn to allow mRNA having poly(A) to be adsorbed on the column. Elution is carried out with water or a hypotonic salt solution such as 10 mM Tris-HCl buffer to isolate the mRNA having poly(A).

Synthesis of cDNA and insertion of the cDNA into a vector are carried out according to the method of Okayama-Berg [Okayama & Berg; Mol. Cell. Biol. $\underline{2}$, 161 (1982)] as described below.

First, a vector primer is synthesized. A vector, e.g. pCDV1, is treated with KpnI in an adequate solution such as a solution consisting of Tris-HCl buffer (e.g. pH 7.5, 10 mM), $MgCl_2$ (e.g. 6 mM) and NaCl (e.g. 10 mM) to cut pCDV1 at KpnI site. The DNA is incubated with terminal deoxynucleotidyltransferase at an appropriate temperature (e.g. 37°C) for an appropriate period (e.g. 20 minutes) in a solution consisting of Tris-HCl buffer (e.g. pH 6.8, 30 mM), sodium cacodylate (e.g. 140 mM), $CaCl_2$ (e.g. 1 mM), dithiothreitol (e.g. 0.1 mM) and dTTP (e.g. 0.25 mM) to add about 60 thymidyl residues to both 3' ends of the vector DNA. Then, the DNA is cut with EcoRI in a solution consisting of Tris-HCl buffer (e.g. pH 7.5, 10 mM), $MgCl_2$ (e.g. 6 mM) and NaCl (e.g. 100 mM). The digested solution is fractionated by low-gelling-temperature agarose gel electrophoresis [Lars Wieslander: Analytical Biochemistry, 98, 305 (1979), hereinafter referred to as LGT method] to recover a DNA fragment of about 3.1 Kb. Then, the DNA is dissolved in an NaCl or KCl hypertonic solution (e.g. 0.5M) and passed through a poly(dA) cellulose column to allow only vector primer molecules having poly(T) to be adsorbed on the column. Elution is carried out with water or a hypotonic salt solution such as 10 mM Tris-HCl buffer to isolate only the vector primer molecules with poly(T).

Then, a linker DNA is synthesized in the following manner. For example, pL1 DNA is treated with PstI in an appropriate solution such as a solution consisting of Tris-HCl buffer (e.g. pH 7.5, 10 mM), $MgCl_2$ (e.g. 6 mM) and NaCl (e.g. 50 mM) to cut pL1 at PstI site. The DNA is treated by the same method as in the synthesis of the vector primer except that dGTP is added in place of dTTP, and oligo dG chains (about 15 mer) are added. The DNA is cut with HindIII in an appropriate solution such as a solution consisting of Tris-HCl buffer (e.g. pH 7.5, 10 mM), $MgCl_2$ (e.g. 6 mM) and NaCl (e.g. 60 mM). A DNA fragment of about 0.5 Kb is fractionated by agarose gel electrophoresis and recovered with DEAE paper. Thus, a linker DNA is obtained.

The thus obtained poly(A)RNA, vector primer and linker DNA are used to synthesize cDNA in the following manner. The poly(A)RNA and vector primer DNA are reacted with a reverse transcriptase at an appropriate temperature (e.g. 37°C) for an appropriate period (e.g. 40 minutes) in a solution consisting of Tris-HCl buffer (e.g. pH 8.3, 50 mM), $MgCl_2$ (e.g. 8 mM), KCl (e.g. 30 mM), dithiothreitol (e.g. 0.3 mM), and dATP, dTTP, dCTP and dGTP (e.g. 2 mM each). Oligo dC chains (about 15 mer) are added to the 3' ends of the thus obtained RNA-DNA double stranded chain under the same conditions as in the case of the addition of dT chains to the vector primer except that dTTP is replaced with dCTP. The DNA is cut with HindIII in a solution consisting of Tris-HCl buffer (e.g. pH 7.5, 10 mM), MgCl$_2$ (e.g. 6 mM) and NaCl (e.g. 60 mM). The previously prepared linker DNA is mixed with the DNA and the mixture is incubated with Escherichia coli DNA ligase at an appropriate temperature (e.g. 12°C) for an appropriate period (e.g. 16 hours) in a solution consisting of Tris-HCl buffer (e.g. pH 7.5, 20 mM), MgCl$_2$ (e.g. 4 mM), (NH$_4$)$_2$SO$_4$ (e.g. 10 mM), KCl (e.g. 0.1M) and β-nicotinamide adenine dinucleotide (β-NAD) (e.g. 0.1 mM) to prepare a ring of the cDNA and linker DNA. To the reaction solution are added 40 μM (final concentration) each dATP, dTTP, dGTP and dCTP. Escherichia coli DNA ligase, Escherichia coli DNA polymerase I and Escherichia coli ribonuclease H are added to replace the RNA part with DNA and to obtain a recombinant plasmid containing a complete double stranded cDNA.

An Escherichia coli strain, e.g. Escherichia coli c600SF8 is transformed with the thus obtained recombinant plasmid, for example, by the method of Scott, et al. [Katsuya Shigesada: Saibo Kogaku (Cell Engineering), 2, 616 (1983)]. Since an ampicillin resistance gene exists in the recombinant plasmid mentioned above, the Escherichia coli transformant is resistant to ampicillin.

Selection of a microorganism strain carrying a new recombinant plasmid DNA having a gene complementary to the mRNA of fish prolactin from the ampicillin-resistant (Ap[R]) strains is generally carried out in the following manner. That is, the transformants obtained above are fixed on a nitrocellulose filter and a synthetic DNA probe having a DNA sequence which is presumed from the amino acid sequence of a known prolactin of Oncorhynchus keta is hybridized thereto to select the transformant showing strong hybridizaion [the method of Grunstein-Hogness, Proc. Natl. Acad. Sci., USA., 72, 3961 (1975)]. The probe DNA is synthesized by a conventional triester method [J. Am. Chem. Soc., 97, 7327 (1975)]. Selection by the synthesized DNA probe is more definitely carried out by the method of Southern, et al. [J. Mol. Biol., 98, 503 (1975)] and a recombinant plasmid DNA having the gene complementary to Oncorhynchus keta prolactin mRNA is identified by the same method.

pSPRL1 is an example of the thus obtained recombinant plasmids. The plasmid can be used as a source of the DNA coding for prolactin of Oncorhynchus keta.

The DNA coding for prolactin of Oncorhynchus keta is cut out from the plasmid carrying the DNA and inserted into a vector DNA. The thus obtained recombinant DNA is incorporated in a microorganism and the obtained transformant is cultured to accumulate a prolactin polypeptide of Oncorhynchus keta in a medium, which is then recovered. In this manner, the prolactin polypeptide of Oncorhynchus keta can be produced.

As the plasmid containing the DNA coding for prolactin of Oncorhynchus keta, pSPRL1 mentioned above is preferably used. Plasmid pSPRL1 can be recovered from Escherichia coli ESPRL1 (FERM BP-776) containing the plasmid by the method of Birnboim [Nucleic Acids Res. 7, 1513 (1979)].

A DNA coding for a prolactin polypeptide derivative is obtained by treating the DNA coding for prolaction of Oncorhynchus keta with an enzyme. Plasmid pSPRL1 is used as a source of the DNA coding for prolactin of Oncorhynchus keta.

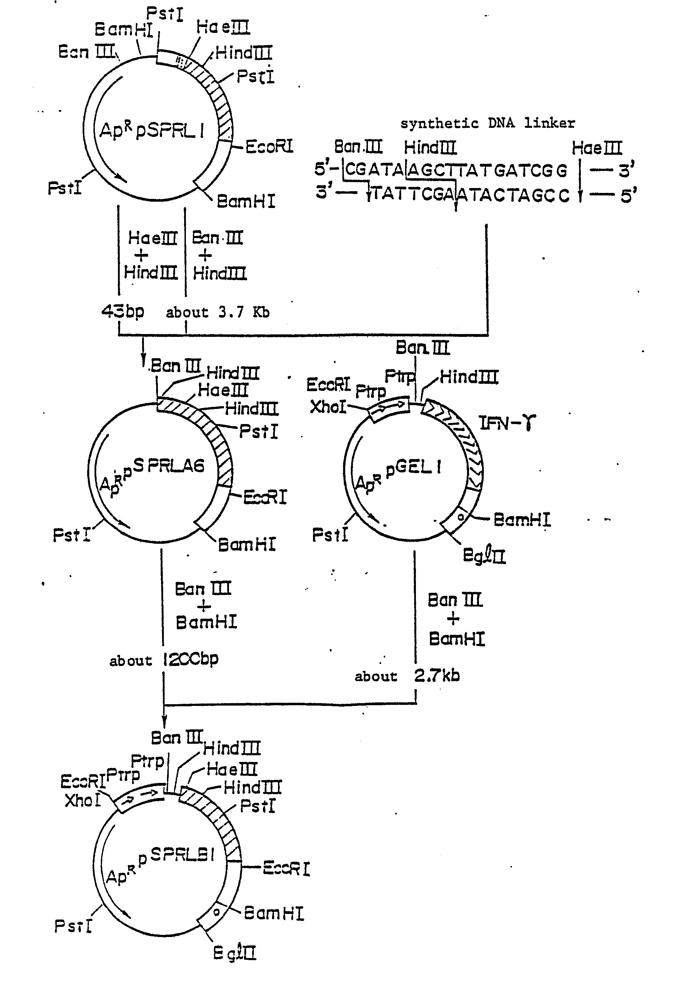

As the vector DNA, any vector can be used so long as the DNA incorporated therein can be expressed in a microorganism. Preferably, a vector DNA wherein a DNA can be inserted downstream from a suitable promoter such as tryptophan (trp) promoter, lactose (lac) promoter or PL promoter and the length between Shine-Dalgarno sequence (referred to as SD sequence hereinafter) and initiation codon (ATG) is adjusted, for example, to 6 - 18 base pairs is employed. Preferred examples of the vector DNA are pGEL1, pTrS20 (Japanese Patent Appliction No. 23319/86) and pKYP26. pGEL1 is a plasmid as illustrated in Fig. 3 and Escherichia coli containing the plasmid was deposited with the Fermentation Research Institute, Agency of Industrial Science and Technology (hereinafter referred to as FRI) as <u>Escherichia coli</u> IGEL1 under FERM BP-629 on October 6, 1984.

Plasmid pTrS20 is obtained according to the method described in Reference Example 1. A microorganism containing plasmid pKYP26 was deposited with the FRI as <u>Escherichia coli</u> IKYP26 (FERM BP-863) on August 8, 1985. Plasmid pKYP26 can be obtained from <u>Escherichia coli</u> IKYP26 (FERM BP-863) containing the plasmid by the method of Birnboim.

Recombination of the DNA coding for the polypeptide and the vector DNA can be carried out by a conventional recombinant DNA techniques wherein both DNAs are digested with restriction enzymes and religated with T4 DNA ligase.

In the case of pSPRL1 and pGEL1 given as an example, two step-construction is carried out as illustrated in Fig. 3. That is, HaeIII-HindIII digestion fragment coding for the vicinity of the N-terminal of mature prolactin peptide of <u>Oncorhynchus keta</u> and HindIII-BanIII digestion fragment containing the remaining cDNA and the vector part are separately prepared from pSPRL1. On the other hand, the synthetic DNA linker as shown below is prepared.

```
       BanIII                                    HaeIII
5' -  |C G A T A A G C T T A T C A T C G G |  - 3'
3' -  |T A T T C G A A T A C T A G C C |     - 5'
                      Met   Ile  Gly
                       1     2    3
```

The DNA fragments and synthetic DNA linker described above are ligated with T4 DNA ligase to obtain the recombinant plasmid pSPRLA6 illustrated in Fig. 3. Then, BanIII-BamHI digestion fragment coding for the mature prolactin peptide of <u>Oncorhynchus keta</u> is obtained from pSPRLA6, and BanIII-BamHI digestion fragment containing a tryptophan promoter is obtained from pGEL1. The two DNA fragments are ligated with T4 DNA ligase to obtain the recombinant plasmid pSPRLB1

illustrated in Fig. 3. The plasmid has a construction wherein a region coding for mature prolactin of <u>Oncorhynchus</u> <u>keta</u> is ligated downstream from the tryptophan promoter.

In the case of prolactin polypeptide derivative, a recombinant plasmid is obtained by the following method.

Plasmid pSPRL1 is subjected to cleavage with KpnI, digestion with Bal31 and cleavage with BamHI to obtain a DNA fragment containing a DNA codig for prolactin polypeptide.

Separately, pKYP26 is cleaved with BamHI and PstI to obtain a DNA fragment of about 1,700 bp containing lpp terminator region. Plasmid pTrS20 is subjected to cleavage with SacI, formation of a flush end with DNA polymerase I and cleavage with PstI to obtain a DNA fragment of about 1,000 bp containing trp promoter.

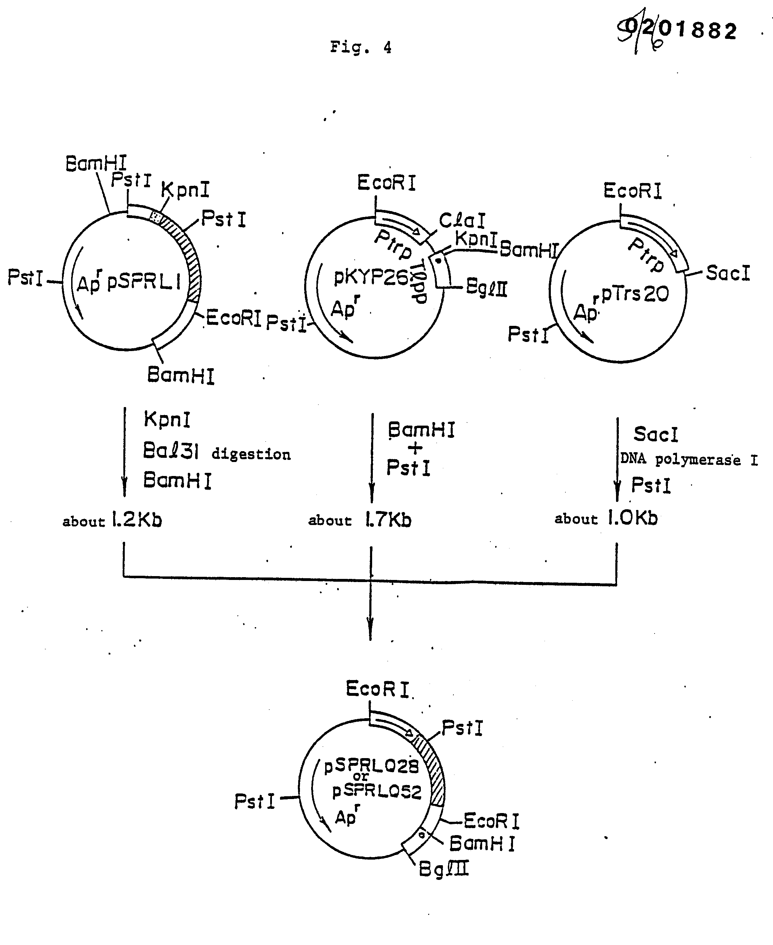

The thus obtained DNAs are combined and a recombinant plasmid is prepared using T4 DNA ligase. <u>Escherichia</u> <u>coli</u> HB101 is transformed with the recombinant plasmid. A colony resistant to ampicillin (Ap$^R$) is obtained and a plasmid DNA is recovered from the colony. Cleavage maps of the thus obtained plasmids pSPRLQ28 and pSPRLQ52 are illustrated in Fig. 4.

Reaction conditions required for the recombinant DNA techniques described above are generally as follows.

Digestion of the DNA with restriction enzymes is usually carried out by reacting 0.1 to 20 µg of the DNA with 0.1 - 100 units, preferably 1 - 3 units of the restriction enzyme per 1 µg of the DNA in a mixture of 2 - 200 mM, preferably 10 - 40 mM Tris-HCl (pH 6.0 - 9.5, preferably pH 7.0 - 8.0), 0 - 200 mM NaCl and 2 - 30 mM, preferably 5 - 10 mM MgCl$_2$ at 20 -70°C (optimal temperature depends on the restriction enzymes used) for 15 minutes to 24 hours. Reaction is usually stopped by heating at 55 - 75°C for 5 - 30 minutes, or alternatively by inactivating the restriction enzyme with a reagent such as phenol or diethylpyrocarbonate.

Purification of the DNA fragments formed by digestion with restriction enzymes is carried out by LGT method or polyacrylamide gel electrophoresis.

Ligation of the DNA fragments is carried out with 0.3 - 10 units of T4 DNA ligase in a mixture of 2 - 200 mM, preferably 10 - 40 mM Tris-HCl (pH 6.1 - 9.5, preferably 7.0 - 8.0), 2 - 20 mM, preferably 5 - 10 mM $MgCl_2$, 0.1 - 10 mM preferably 0.5 - 2.0 mM ATP and 1 - 50 mM, preferably 5 - 10 mM dithiothreitol at 1 - 37°C, preferably 3 - 20°C for 15 minutes to 72 hours, preferably 2 - 20 hours.

The recombinant plasmid DNA formed by the ligation reaction is introduced into Escherichia coli by the transformation method of Cohen, et al. [S.N. Cohen, et al.: Proc. Natl. Acad. Sci. USA, 69, 2110 (1972)], if necessary.

Isolation of the recombinant plasmid DNA from Escherichia coli carrying the DNA is carried out by the method described in Example 1 or the method of Birnboim, et al. [H.C. Birnboim, et al.: Nucleic Acids Res. 7, 1513 (1979)].

Plasmid DNA is digested with 1 - 10 kinds of restriction endonucleases and the cleavage sites are examined by agarose gel electrophoresis or polyacrylamide gel electrophoresis. Further, if necessary, the base sequence of the DNA is determined by the method of Maxam-Gilbert [Proc. Natl. Acad. Sci. 74, 560 (1977)] or the method of Sanger [Sanger, et al.: Proc. Natl. Acad. Sci. USA, 74, 5463 (1977); Amersham Co., M13 cloning and sequencing handbook] using M13 phage.

The fish prolactin polypeptide and derivatives thereof of the present invention are produced by the following method.

That is, Escherichia coli K-12 HB101 is transformed with a plasmid such as pSPRLB1, pSPRLQ28 or pSPRLQ52 and an Escherichia coli strain carrying the plasmid is selected from the ampicillin resistant colonies. The Escherichia coli strain carrying the plasmid is cultured in a medium to produce

- 12 -

0201882

the fish prolactin polypeptide or a derivative thereof in the cultured product.

As the medium, either a synthetic medium or a natural medium can be used so long as it is suitable for the growth of Escherichia coli and the production of the fish prolactin polypeptide.

As the carbon source, glucose, fructose, lactose, glycerol, mannitol, sorbitol, etc. may be used.

As the nitrogen source, $NH_4Cl$, $(NH_4)_2SO_4$, casamino acid, yeast extract, polypeptone, meat extract, Bacto-Tryptone, corn steep liquor, etc. may be used.

In addition, nutrients such as $K_2HPO_4$, $KH_2PO_4$, NaCl, $MgSO_4$, vitamine $B_1$ and $MgCl_2$ may be used.

Culturing is carried out at pH 5.5 - 8.5 and at 18 - 40°C with aeration and stirring.

After culturing for 5 - 90 hours, the prolactin polypeptide of Oncorhynchus keta or the derivative thereof is accumulated in cultured cells. The collected cells are treated with lysozyme, disrupted by repeated freezing and thawing and subjected to centrifugation. The thus obtained supernatant fluid is subjected to extraction according to a conventional method for extraction of polypeptides to recover the polypeptide.

Detection of the polypeptide is carried out by heat-dissolving the cultured cells directly in Sample buffer of Laemmli [Laemmli, Nature, 227, 680 (1970)] and by subjecting the solution to SDS-polyacrylamide gel (the method of Laemmli: the reference mentioned above) and Coomassie Brilliant Blue staining.

Certain specific embodiments of the invention are illustrated by the following representative examples.

Example 1

Preparation of poly(A)RNA from the pituitary gland of
Oncorhynchus keta: ·

An RNA having poly(A) was prepared from the
pituitary gland of Oncorhynchus keta according to guanidium
thiocyanate-cesium chloride method [edited by Maniatis,
et al.: Molecular Cloning, p196, published by Cold Spring
Harbor; Katsuya Shigesada, Saibo Kogaku (Cell Engineering), 2,
616 (1983)] in the following manner.

First, 2g of the freezed pituitary gland of
Oncorhynchus keta (corresponding to about 30 individuals) was
disrupted and solubilized with Teflon homogenizer (5 rpm) in
10 mℓ of a solution consisting of 4M guanidium thiocyanate,
0.5% sarcosine, 5 mM sodium citrate (pH 7) and 0.1M
β-mercaptoethanol. The homogenate was passed through 18G
injector to cut the DNA and put on a layer of 1.2 mℓ each of
5.7M CsCl and 0.1M EDTA (pH 8) in an ultra centrifugation
tube. Centrifugation was carried out at 35,000 rpm for 15
hours using Hitachi RPS40 rotor to recover RNAs as a
precipitate. The RNA precipitate was dissolved in 10 mℓ of
Tris-HCl solution (pH 8.0) containing 1 mM EDTA. After
extraction with phenol-chloroform, the RNA was recovered with
ethanol as a precipitate. About 1 mg of the thus obtained RNA
was dissolved in 1 mℓ of a solution consistig of 10 mM
Tris-HCl (pH 8.0) and 1 mM EDTA. The solution was incubated
at 65°C for 5 minutes and 0.1 mℓ of 5M NaCl was added. The
mixture was subjected to oligo(dT) cellulose column (product
of P-L Biochemicals, column volume 0.5 mℓ) chromatography.
The mRNA having poly(A) adsorbed on the column was eluted with
a solution consisting of 10 mM Tris-HCl (pH 7.5) and 1 mM EDTA
and fractionated in 0.2 mℓ portions to obtain about 10 µg of
the mRNA having poly(A) in the 3rd to 5th fractions.

Example 2

Synthesis of a cDNA and insertion of the DNA into a vector:

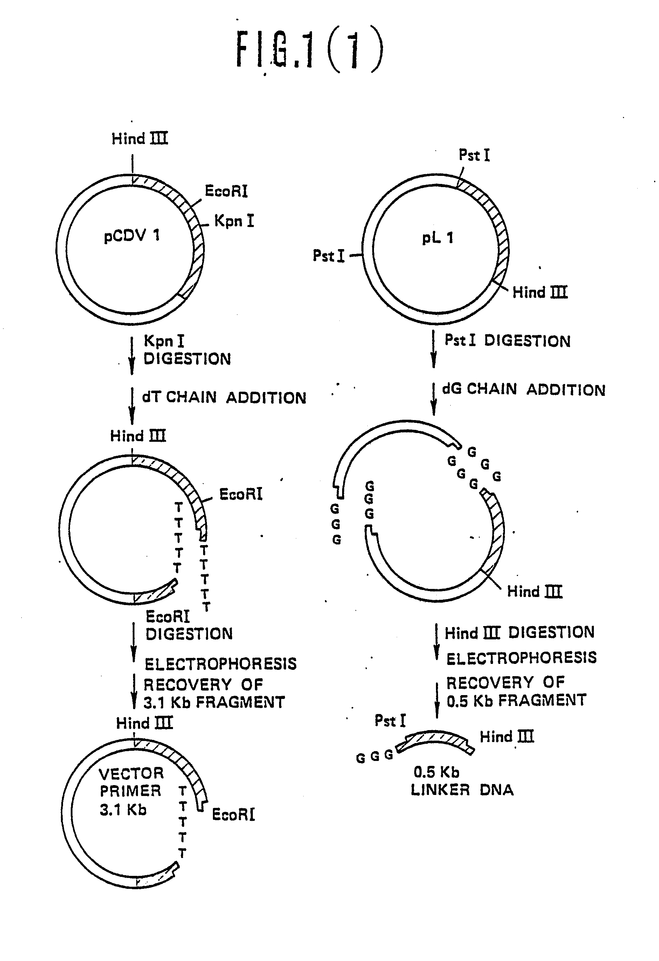

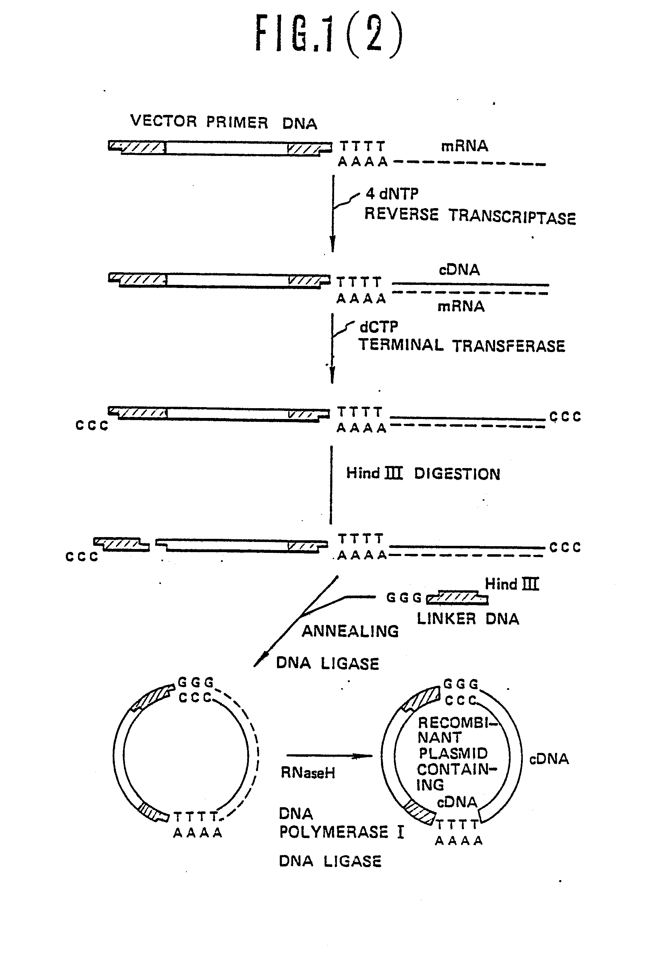

Synthesis of a cDNA and construction of a recombinant plasmid carrying the cDNA were carried out according to the method of Okayama-Berg [Mol. Cell. Biol., 2, 161 (1982)] in the following manner. The process is outlined in Fig. 1.

First, 400 μg of pCDV1 [Okayama & Berg: Mol. Cell. Biol., 3, 280 (1983)] was added to 300 μℓ of a solution consisting of 10 mM Tris-HCl (pH 7.5), 6 mM $MgCl_2$ and 10 mM NaCl, and further 500 units of KpnI (product of Takara Shuzo Co., the restriction enzymes used hereinafter are all products of Takara Shuzo Co., unless otherwise specified) was added. Reaction was carried out at 37°C for 6 hours to cut the plasmid at KpnI site. After phenol-chloroform extraction, a DNA was recovered by ethanol precipitation. About 200 μg of the DNA cut with KpnI was added to 200 μℓ of a solution prepared by adding 0.25 mM dTTP to a buffer (referred to as TdT buffer hereinafter) consisting of 40 mM sodium cacodylate, 30 mM Tris-HCl (pH 6.8), 1 mM $CaCl_2$ and 0.1 mM dithiothreitol (referred to as DTT hereinafter). Further, 81 units of terminal deoxynucleotidyl transferase (referred to as TdT hereinafter) (product of P-L Biochemicals) was added and reaction was carried out at 37°C for 11 minutes, whereby poly(dT) chains (about 67 mer) were added to the 3' ends of pCDV1 cleaved with KpnI. About 100 μg of pCDV1 DNA associated with poly(dT) chains was recovered from the solution by phenol-chloroform extraction and ethanol precipitation. The DNA was added to 150 μℓ of a buffer consisting of 10 mM Tris-HCl (pH 7.5), 6 mM $MgCl_2$ and 100 mM NaCl. Further, 360 units of EcoRI was added and reaction was carried out at 37°C for 2 hours. The reaction product was subjected to LGT method to obtain a DNA fragment of about 3.1 Kb which is about 60 μg of pCDV1 with poly(dT) chains. The DNA was dissolved in 500

µℓ of a solution consisting of 10 mM Tris-HCl (pH 8.0) and 1 mM EDTA. The solution was incubated at 65°C for 5 minutes and 50 µℓ of 5M NaCl was added under ice cooling. The mixture was subjected to oligo(dA) cellulose column (product of Collaborative Research) chromatography. The DNA with enough poly(dT) chains was adsorbed on the column. Elution was carried out with a solution consisting of 10 mM Tris-HCl (pH 8.0) and 1 mM EDTA to obtain 27 µg of pCDV1 with poly(dT) chains (referred to as vector primer hereinafter).

Then, a linker DNA was prepared in the following manner.

About 14 µg of pL1 [Okayama & Berg: Mol. Cell. Biol., 3, 280 (1983)] was added to 200 µℓ of a buffer consisting of 10 mM Tris-HCl (pH 7.5), 6 mM MgCl$_2$ and 50 mM NaCl. Further, 50 units of PstI was added and reaction was carried out at 37°C for 4 hours to cut pL1 DNA at PstI site. The reaction product was subjected to phenol-chloroform extraction and ethanol precipitation to recover about 13 µg of pL1 DNA cleaved with PstI. About 13 µg of the DNA was added to 50 µℓ of TdT buffer containing 0.25 mM (final concentration) dGTP. Further, 54 units of TdT (product of P-L Biochemicals) was added and incubation was carried out at 37°C for 13 minutes to add (dG) chains (about 14 mer) to the 3' ends of pL1 cut with PstI. A DNA was recovered by phenol-chloroform extraction and ethanol precipitation. The DNA was added to 100 µℓ of a buffer consisting of 10 mM Tris-HCl (pH 7.5), 6 mM MgCl$_2$ and 60 mM NaCl. Further, 80 units of HindIII was added and incubation was carried out at 37°C for 3 hours to cut pL1 DNA at HindIII site. The reaction product was fractionated by agarose gel electrophoresis and a DNA fragment of about 0.5 Kb was recovered by DEAE paper method [Dretzen, et al., Anal. Biochem., 112, 295 (1981)]. The DNA was linker DNA with oligo(dG) chain (referred to as linker-DNA hereinafter).

About 2 µg of the poly(A)RNA and about 1.4 µg of the vector primer prepared above were dissolved in 22.3 µℓ of a solution consisting of 50 mM Tris-HCl (pH 8.3), 8 mM $MgCl_2$, 30 mM KCl, 0.3 mM DTT, 2 mM dNTP (dATP, dTTP, dGTP and dCTP) and 10 units of ribonuclease inhibitor (product of P-L Biochemicals). Ten units of reverse transcriptase (product of Seikagaku Kogyo Co.) was added and incubation was carried out at 37°C for 40 minutes to synthesize a DNA complementary to the mRNA. The reaction product was subjected to phenol-chloroform extraction and ethanol precipitation to recover a vector-primer DNA associated with RNA-DNA double stranded chain. The DNA was dissolved in 20 μℓ of TdT buffer containing 66 μM dCTP and 0.2 μg of poly(A). Fourteen units of TdT (product of P-L Biochemicals) was added and incubation was carried out at 37°C for 8 minutes to add (dC) chains (12 mer) to the 3' ends of the cDNA. The reaction product was subjected to phenol-chloroform extraction and ethanol precipitation to recover a cDNA-vector primer DNA associated with (dC) chains. The DNA was dissolved in 400 μℓ of a solution consisting of 10 mM Tris-HCl (pH 7.5), 6 mM $MgCl_2$ and 60 mM NaCl. Twenty units of HindIII was added and incubation was carried out at 37°C for 2 hours to cut the DNA at HindIII site. The reaction product was subjected to phenol-chloroform extraction and ethanol precipitation to obtain 0.5 pmole of a cDNA-vector primer DNA associated with (dC) chains. Then, 0.08 pmole of the DNA and 0.16 pmole of the linker-DNA mentioned above were dissoved in 40 μℓ of a solution consisting of 10 mM Tris-HCl (pH 7.5), 0.1M NaCl and 1 mM EDTA and incubations were carried out at 65°C, 42°C and 0°C for 10 minutes, 25 minutes and 30 minutes, respectively. The reaction solution was adjusted to 400 μℓ (total volume) of a solution having a composition of 20 mM Tris-HCl (pH 7.5), 4 mM $MgCl_2$, 10 mM $(NH_4)_2SO_4$, 0.1M KCl and 0.1 mM β-NAD. Ten units of Escherichia coli DNA ligase (product of New England Biolabs) was added to the reaction solution and incubation was carried out at 11°C overnight. The reaction solution was adjusted to a solution containing 40 μM dNTP and 0.15 mM

β-NAD.  Five units of Escherichia coli DNA ligase, 7 units of Escherichia coli DNA polymerase I (product of P-L Biochemicals) and 2 units of Escherichia coli ribonuclease H (product of P-L Biochemicals) were added and incubation was carried out at 12°C for one hour and successively at 25°C for one hour.  By the reaction mentioned above, a recombinant DNA containing the cDNA was cyclized, the RNA part of the RNA-DNA double stranded chain was replaced with the DNA and a recombinant plasmid having complete double stranded DNA was formed.

Example 3

Selection of a recombinant DNA containing Oncorhynchus keta prolactin cDNA:

Escherichia coli c600SF8 [Cameron:  Proc. Natl. Acad. Sci. USA, 72, 3416 (1975)] was transformed using the recombinant plasmid obtained in Example 2 by the method of Scott, et al. [Katsuya Shigesada:  Saibo Kogaku (Cell Engineering) 2, 616 (1983)].  About 1,700 colonies thus obtained were fixed on nitrocellulose.  Seven strains were selected which hybridized strongly at 42°C with the probe wherein a synthetic DNA corresponding to the 122nd to 127th amino acid sequence from the N-terminal of the Oncorhynchus keta prolactin, i.e.

```
         1 2 3 4 5  6  7  8  9  10 11 12 13 14 15 16 17
                                                         17
     5'  A T G G T  A  A  A  T  A  A  A  A  T  G  G  G  3'  mer
            (T)(G)     (C)          (G)
            (G)
            (C)
```

(the 6th base is one of A, T, G and C, the 7th is A or G, the 9th is T or C, the 12th is A or G and combination of the bases makes 32 kinds of synthetic DNAs) was labelled with $^{32}P$ [the method of Grunstein-Hogness, Proc. Natl. Acad. Sci., USA, 72, 3961 (1975)].  It was confirmed by the method of Southern [J. Mol. Biol., 98, 503 (1975)] that the 7 strains hybridized with the probe mentioned above.  The plasmids named pSPRL1, 2, 4, 7, 8, 10 and 11 respectively have the DNA sequence presumed from the amino acid sequence of the <u>Oncorhvnchus keta</u> prolactin and are considered to contain a prolactin cDNA.

Example 4

The base sequence of plasmid pSPRL1:

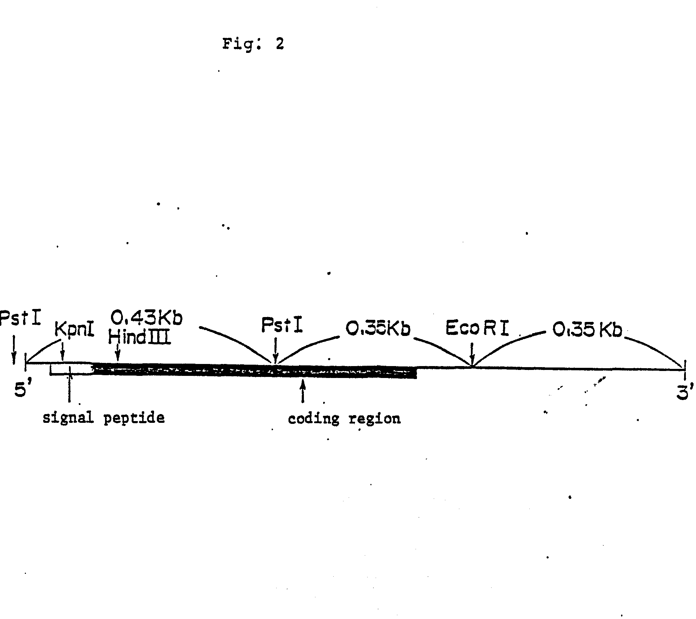

The 7 plasmids obtained above were digested with various restriction endonucleases and cleavage maps of the cDNA parts were determined.  The restriction endonuclease map of the cDNA in plasmid pSPRL1 (hereinafter referred to as SPRL1) is illustrated in Fig. 2.

pSPRL1 which hybridizes most strongly with the synthetic DNA probe described in Example 3 and which is considered to contain the cDNA having an almost complete length was examined by the method of Sanger using M13 phage [Sanger, <u>et al</u>.; Proc. Natl. Acad. Sci., USA, <u>74</u>, 5463 (1977): Amersham, M13 cloning and sequencing handbook] to determine the whole nucleotide sequence of the translational region. The nucleotide sequence is illustrated in Table 1.

In Table 1, the base numbers 1-69 code for signal peptide and 70-630 code for the mature peptide of <u>Oncorhynchus keta</u> prolactin.

It was confirmed that the amino acid sequence assumed from the cDNA sequence in pSPRL1 coincides with a part of the amino acid sequence determined from the natural <u>Oncorhynchus keta</u> prolactin and that the cDNA codes for <u>Oncorhvnchus keta</u> prolactin.  <u>Escherichia coli</u> containing pSPRL1 was deposited with the Fermentation Research Institute, Agency of Industrial Science and Technology (FRI) as <u>Escherichia coli</u> ESPRL1 (FERM BP-776) on April 26, 1985.

## Table 1

```
          10        20          30        40        50        60
ATGGCTCGCCGATCCCAGGGTACCAAACTCCACTTAGCAGTTCTGTGTCTAGTTGTGTCC
MetAlaArgArgSerGlnGlyThrLysLeuHisLeuAlaValLeuCysLeuValValSer

          70        80          90       100       110       120
TGTCATGCCATTGGCCTTAGTGACCTAATGGAGAGAGCTTCCCAGCGATCAGACAAGCTT
CysHisAlaIleGlyLeuSerAspLeuMetGluArgAlaSerGlnArgSerAspLysLeu

         130       140         150       160       170       180
CACTCACTCAGCACTTCCCTCAACAAGGACCTGGACTCTCACTTCCCACCAATGGGACGA
HisSerLeuSerThrSerLeuAsnLysAspLeuAspSerHisPheProProMetGlyArg

         190       200         210       220       230       240
GTGATGATGCCACGTCCATCTATGTGTCACACCTCCTCACTCCAGATACCCAAGGACAAG
ValMetMetProArgProSerMetCysHisThrSerSerLeuGlnIleProLysAspLys

         250       260         270       280       290       300
GAGCAAGCGCTTAGAGTATCGGAGAATGAGCTGATCTCCCTGGCTCGCTCCCTCCTGCTG
GluGlnAlaLeuArgValSerGluAsnGluLeuIleSerLeuAlaArgSerLeuLeuLeu

         310       320         330       340       350       360
GCCTGGAATGATCCCCTGCTGCTGCTCTCCTCTGAGGCGCCCACTCTGCCACACCCCTCC
AlaTrpAsnAspProLeuLeuLeuLeuSerSerGluAlaProThrLeuProHisProSer

         370       380         390       400       410       420
AATGGTGACATCAGCAGTAAGATCAGGGAACTGCAGGACTACTCCAAGAGCCTAGGAGAC
AsnGlyAspIleSerSerLysIleArgGluLeuGlnAspTyrSerLysSerLeuGlyAsp

         430       440         450       460       470       480
GGACTGGACATACTGGTCAACAAGATGGGCCCCTCCTCCCAGTACATTTCTTCAATCCCC
GlyLeuAspIleLeuValAsnLysMetGlyProSerSerGlnTyrIleSerSerIlePro

         490       500         510       520       530       540
TTCAAGGGTGGAGACCTCGGCAATGACAAGACCTCCCGCCTCATCAACTTCCACTTCCTT
PheLysGlyGlyAspLeuGlyAsnAspLysThrSerArgLeuIleAsnPheHisPheLeu

         550       560         570       580       590       600
ATGTCCTGCTTCCGCAGGGACTCCCACAAAATCGACAGTTTCCTCAAGGTCCTTCGATGC
MetSerCysPheArgArgAspSerHisLysIleAspSerPheLeuLysValLeuArgCys

         610       620         630
CGGGCTACCAAAATGCGACCAGAAACATGTTAG
ArgAlaThrLysMetArgProGluThrCys***
```

- 20 -

0201382

Example 5

Construction of recombinant plasmid coding for Oncorhynchus keta prolactin:

(1) Construction of recombinant plasmid pSPRLB1 coding for the mature prolactin of Oncorhynchus keta from pSPRL1:

In this example, 10 µg of plasmid pSPRL1 containing a DNA coding for prolactin of Oncorhynchus keta was dissolved in 100 µℓ of a solution containing 10 mM Tris-HCl (pH 7.5), 7 mM MgCl$_2$, and 100 mM NaCl (referred to as Y-100 buffer solution hereinafter). Then, 30 units of restriction enzyme HindIII (product of Toyo Boseki Co.) was added and digestion reaction was carried out at 37°C for 2 hours. About 1 µg of a DNA fragment of about 0.7 Kb containing N-terminal region, 5'-non-translational region of the prolactin and the vector part encoded by pSPRL1 was obtained from the reaction solution by LGT method. The DNA of 0.7 Kb was dissolved in 20 µℓ Y-100 buffer solution and 8 units of HaeIII was added. Digestion reaction was carried out at 37°C for 2 hours. About 0.1 µg of a DNA fragment of 43 bp corresponding to N-terminal region was obtained from the reaction solution by polyacrylamide gel electrophoresis and filtration with glass filter.

Then, 2 µg of pSPRL1 was dissolved in 30 µℓ of Y-100 buffer solution. Eight units of BanIII and 16 units of HindIII were added and digestion reaction was carried out at 37°C for 2 hours. About 1.0 µg of a DNA fragment of about 3.7 Kb containing C-terminal region, 3'-non-translational region of Oncorhynchus keta prolactin and vector part encoded by pSPRL1 was obtained from the reaction solution by LGT method.

In order to add a translational initiation codon ATG necessary for the expression of the DNA coding for the mature prolactin of Oncorhynchus keta and to ligate a vector DNA and the DNA mentioned above, a DNA linker set forth below was synthesized.

```
    BanIII                              HaeIII
5' -│C G A T A A G C T T│A T G│A T C G G │- 3'   18 mer
3' -│  T A T T C G A A│T A C│T A G C C ↓ - 5'   16 mer
                        Met   Ile   Gly
```

Two single stranded DNAs of 18 mer and 16 mer were synthesized by a conventional triester method [R. Crea, et al.: Proc. Natl. Acad. Sci., USA, 75, 5765 (1978)]. Then, 40 pmoles each of the 18 mer and 16 mer DNAs were dissolved in 20 μℓ of a solution consisting of 50 mM Tris-HCl (pH 7.5), 10 mM MgCl$_2$, 10 mM DTT, and 1 mM ATP. Six units of T4 polynucleotide kinase (product of Takara Shuzo Co.) was added and phosphorylation reaction was carried out at 37°C for 30 minutes.

Then, 0.08 pmole of the HaeIII-HindIII fragment (43 bp) of pSPRL1 and 0.02 pmole of the BanIII-HindIII fragment (about 3.7 Kb) of pSPRL1 obtained above were dissolved in 30 μℓ of a solution consisting of 50 mM Tris-HCl (pH 7.5), 10 mM MgCl$_2$, 10 mM DTT and 1 mM ATP. Five μℓ of the synthetic DNA phosphorylation reaction solution obtained above was added. Six units of T4 DNA ligase (product of Takara Shuzo Co.) was added to the mixture and ligation reaction was carried out at 4°C for 18 hours.

Escherichia coli K294 was transformed using the reaction solution to obtain an Ap$^R$ colony. A plasmid DNA pSPRLA6 illustrated in Fig. 3 was recovered from the colony. The structure of pSPRLA6 was recognized by the cleavage with PstI, BamHI, EcoRI, BanIII, BglII and HindIII and agarose gel electrophoresis.

(2)  Insertion of a region coding for the mature prolactin of Oncorhynchus keta in pSPRLA6 into an expression vector pGEL1:

In this step, 2 μg of pSPRLA6 was dissolved in 30 μℓ of Y-100 buffer solution and 8 units each of BamHI and BanIII were added. Digeston reaction was carried out at 37°C for 3 hours. About 0.1 μg of a DNA fragment of about 1.2 Kb coding for the mature prolactin of Oncorhynchus keta was obtained from the reaction solutin by LGT method.

Separately, 2 µg of pGEL1 was dissolved in 40 µl of Y-100 buffer solution and 10 units each of BamHI and BanIII were added. Digestion reaction was carried out at 37°C for 3 hours. About 0.2 µg of a DNA fragment of about 2.7 Kb containing a tryptophan promoter was obtained from the reaction solution by LGT method.

Then, 0.01 µg of the BanIII-BamHI fragment (about 1.2 Kb) of pSPRLA6 and 0.015 µg of the BanIII-BamHI fragment (about 2.7 Kb) of pGEL1 obtained above were dissolved in 20 µl of a solution consisting of 50 mM Tris-HCl (pH 7.5), 10 mM MgCl$_2$, 10 mM DTT and 1 mM ATP and 6 units of T4 DNA ligase was added. Ligation reaction was carried out at 4°C for 18 hours.

Escherichia coli K294 was transformed using the reaction solution to obtain an Ap$^R$ colony. A plasmid DNA pSPRLB1 illustrated in Fig. 3 was recovered from the colony. The structure of pSPRLB1 was recognized by the cleavage with EcoRI, HindIII, BanIII, PstI and BamHI and agarose gel electrophoresis.

Example 6

Production of prolactin peptide of Oncorhynchus keta by Escherichia coli containing pSPRLB1:

Escherichia coli W3110 strA strain (FERM BP-732) was transformed with the recombinant plasmid pSPRLB1 obtained in Example 5 by a conventional method. An Ap$^R$ colony obtained was inoculated in 8 ml of MCG medium (pH 7.2) consisting of 0.6% Na$_2$HPO$_4$, 0.3% KH$_2$PO$_4$, 0.5% NaCl, 0.1% NH$_4$Cl, 0.5% glucose, 0.5% casamino acid, 1 mM MgSO$_4$ and 4 µg/ml vitamine B1 and culturing was carried out at 30°C for 18 hours. The culture broth was centrifuged at 8,000 rpm for 10 minutes to recover cells. The cells were suspended in Sample buffer of Laemmli and subjected to SDS-polyacrylamide gel electrophoresis and Coomassie Brilliant Blue staining to detect a polypeptide band at the portion of a molecular weight of about 27,000. The band was not observed in the case of using Escherichia coli which does not contain the plasmid. As the result, it was confirmed that Escherichia coli carrying pSPRLB1 produced prolactin polypeptide of Oncorhynchus keta in a large amount.

Escherichia coli containing plasmid pSPRLB1 was deposited with the FRI as Escherichia coli ESPRLB1 under FERM BP-777 on April 26, 1985.

Example 7

Construction of a plasmid for expressing prolactin polypeptide derivative of Oncorhynchus keta:

In this step, 6 μg of plasmid pSPRL1 containing a DNA coding for prolactin polypeptide of Oncorhynchus keta was dissolved in 50 μℓ of a solution consisting of 10 mM Tris-HCl (pH 7.5), 7 mM $MgCl_2$ and 6 mM mercaptoethanol (referred to as Y-0 buffer solution hereinafter) and 15 units of KpnI was added. Digestion reaction was carried out at 37°C for 3 hours.

An equal amount of phenol saturated with 10 mM Tris-HCl (pH 7.5) and 1 mM EDTA was added and the mixture was stirred vigorously, followed by low speed centrifugation (3,300 rpm, 10 minutes, the same conditions are employed hereinafter) to recover the water layer. This step is referred to as phenol extraction method.

Then, an equal amount of chloroform was added and the mixture was stirred vigorously, followed by low speed centrifugation. This step is referred to as chloroform extraction method.

One tenth volume of 3M sodium acetate was added and 2.5 times volume of ethanol was added. The mixture was allowed to stand at -20°C for 2 hours and a precipitate was recovered by cooling centrifugation (4°C, 11,000 rpm, 10 minutes). This step is referred to as ethanol precipitation method.

The precipitate was dissolved in 50 µℓ of a solution consisting of 20 mM Tris-HCl (pH 8.0), 12 mM CaCl$_2$, 12 mM MgCl$_2$, 200 mM NaCl and 1 mM EDTA. The solution was kept at 20°C for 5 minutes. Then, 10 units of BAL31 nuclease (product of Bethesda Research Laboratories Inc.) was added and decomposition reaction was carried out at 20°C for 20 minutes. After the reaction, phenol extraction and chloroform extraction were carried out and a DNA was recovered by ethanol precipitation. The DNA was dissolved in 40 µℓ of a solution consisting of 10 mM Tris-HCl (pH 7.5), 7mM MgCl$_2$, 100 mM NaCl and 6 mM mercaptoethanol (referred to as Y-100-2 buffer solution hereinafter) and 10 units of BamHI was added. Digestion reaction was carried out at 37°C for 2 hours. From the reaction solution, about 0.5 µg of a DNA fragment of about 1.2 Kb containing a DNA coding for Oncorhynchus keta prolactin polypeptide was obtained by LGT method.

Separately, 3 µg of pKYP26 was dissolved in 40 µℓ of Y-100-2 buffer solution, and 8 units of BamHI and 10 units of PstI were added. Digestion reaction was carried out at 37°C for 2 hours. From the reaction solution, 0.3 µg of a DNA fragment of about 1,700 base pairs (bp) containing lpp terminator part was obtained by LGT method.

Separately, 3 µg of pTrS20 was dissolved in 40 µℓ of Y-0 buffer solution. 10 units of SacI was added and digestion reaction was carried out at 37°C for 2 hours. After phenol extraction and chloroform extraction of the reaction solution, a DNA was recovered by ethanol precipitation. The DNA was dissolved in 30 µℓ of a solution consisting of 50 mM Tris-HCl (pH 7.5), 5 mM MgCl$_2$, 10 mM mercaptoethanol and 0.4 mM each of dATP, dCTP, dGTP and dTTP. 4 units of DNA polymerase I (product of Takara Shuzo Co.) was added and reaction was carried out at 20°C for one hour. After phenol extraction and chloroform extraction of the reaction solution, a DNA was recovered by ethanol precipitation. The DNA was dissolved in 30 µℓ of Y-100-2 buffer solution and 6 units of PstI was added. Digestion reaction was carried out at 37°C for 2 hours. From the reaction solution, 0.1 µg of a DNA fragment of about 1,000 bp containing a trp promoter was obtained by LGT method.

About 0.2 µg of the DNA fragment (about 1,200 bp) derived from pSPRL1, about 0.05 µg of the DNA fragment (about 1,700 bp) derived from pKYP26 and about 0.05 µg of the DNA fragment (about 1,000 bp) derived from pTrS20, which were obtained above, were dissolved in 30 µℓ of a solution consistig of 20 mM Tris-HCl (pH 7.5), 10 mM $MgCl_2$, 10 mM dithiothreitol and 1 mM ATP (referred to as T4 DNA ligase buffer solution hereinafter). 3 units of T4 DNA ligase (product of Takara Shuzo Co.) was added and ligation reaction was carried out at 4°C for 16 hours.

*Escherichia coli* HB101 [Bolivar et. al., Gene, *2*, 75 (1977)] was transformed using the reaction solution and colonies showing $Ap^R$ were obtained. Plasmid DNAs pSPRLQ28 and pSPRLQ52 illustrated in Fig. 4 were recovered from the colonies by the method of Birnboim, et. al. [Nucleic Acids Research, *7*, 1513 (1979)]. Nucleotide sequences of these plasmid DNAs were determined to make clear that pSPRLQ28 had a structure coding for a prolactin polypeptide derivative wherein the DNA coding for four amino acids at the N-terminal of *Oncorhynchus keta* prolactin polypeptide was lost and methionine derived from pTrS20 was attached to the N-terminal amino acid (aspartic acid) of the remaining polypeptide. Similarly, it was made clear that pSPRLQ52 had a structure coding for a prolactin polypeptide derivative wherein the DNA coding for nine amino acids at the N-terminal was lost and methionine derived from pTrS20 was attached to the N-terminal amino acid (alanine) of the remaining polypeptide.

Nucleotide sequences of the genes of *Oncorhynchus keta* prolactin polypeptide derivatives in pSPRLQ28 and pSPRLQ52 and amino acid sequences of the polypeptide derivatives are illustrated in Table 2 and Table 3, respectively.

0201882

## Table 2

### - Genetic Code [Universal]

```
        10        20        30        40        50        60
ATGGACCTAATGGAGAGAGCTTCCCAGCGATCAGACAAGCTTCACTCACTCAGCACTTCC
MetAspLeuMetGluArgAlaSerGlnArgSerAspLysLeuHisSerLeuSerThrSer

        70        80        90       100       110       120
CTCAACAAGGACCTGGACTCTCACTTCCCACCAATGGGACGAGTGATGATGCCACGTCCA
LeuAsnLysAspLeuAspSerHisPheProProMetGlyArgValMetMetProArgPro

       130       140       150       160       170       180
TCTATGTGTCACACCTCCTCACTCCAGATACCCAAGGACAAGGAGCAAGCGCTTAGAGTA
SerMetCysHisThrSerSerLeuGlnIleProLysAspLysGluGlnAlaLeuArgVal

       190       200       210       220       230       240
TCGGAGAATGAGCTGATCTCCCTGGCTCGCTCCCTCCTGCTGGCCTGGAATGATCCCCTG
SerGluAsnGluLeuIleSerLeuAlaArgSerLeuLeuLeuAlaTrpAsnAspProLeu

       250       260       270       280       290       300
CTGCTGCTCTCCTCTGAGGCGCCCACTCTGCCACACCCCTCCAATGGTGACATCAGCAGT
LeuLeuLeuSerSerGluAlaProThrLeuProHisProSerAsnGlyAspIleSerSer

       310       320       330       340       350       360
AAGATCAGGGAACTGCAGGACTACTCCAAGAGCCTAGGAGACGGACTGGACATACTGGTC
LysIleArgGluLeuGlnAspTyrSerLysSerLeuGlyAspGlyLeuAspIleLeuVal

       370       380       390       400       410       420
AACAAGATGGGCCCCTCCTCCCAGTACATTTCTTCAATCCCCTTCAAGGGTGGAGACCTC
AsnLysMetGlyProSerSerGlnTyrIleSerSerIleProPheLysGlyGlyAspLeu

       430       440       450       460       470       480
GGCAATGACAAGACCTCCCGCCTCATCAACTTCCACTTCCTTATGTCCTGCTTCCGCAGG
GlyAsnAspLysThrSerArgLeuIleAsnPheHisPheLeuMetSerCysPheArgArg

       490       500       510       520       530       540
GACTCCCACAAAATCGACAGTTTCCTCAAGGTCCTTCGATGCCGGGCTACCAAAATGCGA
AspSerHisLysIleAspSerPheLeuLysValLeuArgCysArgAlaThrLysMetArg

       550
CCAGAAACATGTTAG
ProGluThrCys***
```

Table 3

- Genetic Code [Universal]

```
        10        20        30        40        50        60
ATGGCTTCCCAGCGATCAGACAAGCTTCACTCACTCAGCACTTCCCTCAACAAGGACCTG
MetAlaSerGlnArgSerAspLysLeuHisSerLeuSerThrSerLeuAsnLysAspLeu

        70        80        90       100       110       120
GACTCTCACTTCCCACCAATGGGACGAGTGATGATGCCACGTCCATCTATGTGTCACACC
AspSerHisPheProProMetGlyArgValMetMetProArgProSerMetCysHisThr

       130       140       150       160       170       180
TCCTCACTCCAGATACCCAAGGACAAGGAGCAAGCGCTTAGAGTATCGGAGAATGAGCTG
SerSerLeuGlnIleProLysAspLysGluGlnAlaLeuArgValSerGluAsnGluLeu

       190       200       210       220       230       240
ATCTCCCTGGCTCGCTCCCTCCTGCTGGCCTGGAATGATCCCCTGCTGCTGCTCTCCTCT
IleSerLeuAlaArgSerLeuLeuLeuAlaTrpAsnAspProLeuLeuLeuLeuSerSer

       250       260       270       280       290       300
GAGGCGCCCACTCTGCCACACCCCTCCAATGGTGACATCAGCAGTAAGATCAGGGAACTG
GluAlaProThrLeuProHisProSerAsnGlyAspIleSerSerLysIleArgGluLeu

       310       320       330       340       350       360
CAGGACTACTCCAAGAGCCTAGGAGACGGACTGGACATACTGGTCAACAAGATGGGCCCC
GlnAspTyrSerLysSerLeuGlyAspGlyLeuAspIleLeuValAsnLysMetGlyPro

       370       380       390       400       410       420
TCCTCCCAGTACATTTCTTCAATCCCCTTCAAGGGTGGAGACCTCGGCAATGACAAGACC
SerSerGlnTyrIleSerSerIleProPheLysGlyGlyAspLeuGlyAsnAspLysThr

       430       440       450       460       470       480
TCCCGCCTCATCAACTTCCACTTCCTTATGTCCTGCTTCCGCAGGGACTCCCACAAAATC
SerArgLeuIleAsnPheHisPheLeuMetSerCysPheArgArgAspSerHisLysIle

       490       500       510       520       530       540
GACAGTTTCCTCAAGGTCCTTCGATGCCGGGCTACCAAAATGCGACCAGAAACATGTTAG
AspSerPheLeuLysValLeuArgCysArgAlaThrLysMetArgProGluThrCys***
```

Example 8

Production of prolactin derivative polypeptide of Oncorhynchus keta by Escherichia coli containing pSPRLQ28 or pSPRLQ52:

Escherichia coli W3110 strA strain (FERM BP-732) was transformed with the recombinant plasmid pSPRLQ28 or pSPRLQ52 obtained in Example 7 by a conventional method. An Ap$^R$ colony obtained was inoculated in MCG medium (pH 7.2) consisting of 0.6% $Na_2HPO_4$, 0.3% $KH_2PO_4$, 0.5% NaCl, 0.1% $NH_4Cl$, 0.5% glucose, 0.5% casamino acid, 1 mM $MgSO_4$ and 4 µg/mℓ vitamine B1 and containing 50 µg/mℓ ampicillin and 25 µg/mℓ tryptophan, and culturing was carried out with shaking at 30°C. When OD 600 reached 1.0, 20 µg/mℓ indolylacrylic acid was added and culturing with shaking was cotinued at 30°C for 3 hours. The culture broth was centrifuged at 8,000 rpm for 10 minutes to recover cells. The cells were suspended in Sample buffer of Laemmli and subjected to SDS-polyacrylamide gel electrophoresis and Coomassie Brilliant Blue staining to detect a polypeptide band at the portion of a molecular weight of about 26,000 in the case of Escherichia coli containing pSPRLQ28 and about 25,000 in the case of Escherichia coli containing pSPRLQ52. The bands were not observed in the case of using Escherichia coli which does not contain the plasmids. Further, after SDS-polyacrylamide gel electrophoresis, Western Blotting was carried out by the method of Towbin, et al. [Towbin, H., et al., Proc. Natl. Acad. Sci., USA, 76, 4350 - 4354 (1979)]. The polypeptides having the molecular weights of about 26,000 and 25,000 mentioned above reacted specifically with anti Oncorhynchus keta antibody. As the result, it was confirmed that Escherichia coli carrying pSPRLQ28 or pSPRLQ52 produced prolactin polypeptide derivative of Oncorhynchus keta in a large amount.

Escherichia coli containing plasmids pSPRLQ28 and pSPRLQ52 were deposited with the FRI as Escherichia coli ESPRLQ28 (FERM BP-1006) and Escherichia coli ESPRLQ52 (FERM BP-1007) on March 28, 1986.

Example 9

The microorganisms which produce prolactin polypeptide derivatives of Oncorhynchus keta were cultured according to Example 8. The cells were collected by centrifugation at 8,000 rpm for 10 minutes and washed with a buffer solution (pH 7.5) containing 30 mM NaCl and 30 mM Tris-HCl. The washed cells were suspended in 5 ml of the buffer mentioned above and subjected to ultrasonic disintegration (Branson Sonic Power Company, Sonifier cell disruptor 200, output cotrol treatment for 2 and 10 minutes). Disrupted cells were recovered by centrifugation at 15,000 rpm for 30 minutes. The disrupted cells were treated according to the method of Marston, et al. [F.A.O. Marston, et al.: Biotechnology, 2, 800 (1984)], whereby prolactin polypeptide derivative of Oncorhynchus keta was extracted, purified, solubilized and renatured. Growth promoting activity of the prolactin polypeptide derivative of Oncorhynchus keta was determined by the following method.

Determination of activity of Oncorhynchus keta prolactin:

Fry of rainbow trout weighing an average of 12g each were divided to groups each consisting of 10 individuals and cultivated in a cycle system tank at a water temperature of 15°C. Mixed feed (Masu 4C, product of Nippon Haigo Shiryo Co.) was given in an amount of 4% of body weight divided in two portions per day for 9 days and thereafter 3%. Prolactin polypeptide derivative of Oncorhynchus keta was dissolved in a small amount of 0.01N sodium hydroxide aqueous solution and 0.9% sodium chloride aqueous solution was added to make the concentration of the prolactin polypeptide derivative 1 µg/5 µl. The sample solution was injected intraperitoneally in an amount of 1 µg/1g weight. 0.9% sodium chloride aqueous solution was administered to a control group. Injection was carried out five times at an interval of five days and body weight was simultaneously measured. As the result, it was confirmed that the prolactin polypeptide derivatives encoded by plasmids pSPRLQ28 and pSPRLQ52 of the present invention promoted the growth of rainbow trout.

Reference Example 1

Construction of ATG vector pTrS20:

ATG vector pTrS20 wherein the distance between SD sequence and ATG initiation codon is 14 bases and SacI site is present immediately after ATG codon was constructed.

3 µg of pKYP10 prepared by the method described in Japanese Unexamined Published Patent Application 110600/83 was dissolved in 30 µℓ of Y-100-2 buffer solution and 6 units each of BanIII and NruI (products of New England Biolabs) were added. Cleavage reaction was carried out at 37°C for 3 hours. About 0.5 µg of a DNA fragment (BanIII-NruI fragment) of about 3.8 Kb containing Ptrp was obtained from the reaction solution by LGT method.

Separately, in order to add ATG initiation codon downstream from Ptrp, the DNA linker shown below was synthesized by the triester method [R. Crea, et al.: Proc. Natl. Acad. Sci., USA, 75, 5765 (1978)].

```
          BanIII      HindIII                        SacI   NruI
                                    M e t
    5' - C G A T A A G C T T A T G A G C T C G - 3'  (19-mer)
         3'- T A T T C G A A T A C T C G A G C - 5'  (17-mer)
```

Synthetic DNAs of 19-mer and 17-mer (10 pmoles each) were dissolved in 20 µℓ (total volume) of a solution containing 50 mM Tris-HCl (pH 7.5), 10 mM $MgCl_2$, 5 mM dithiothreitol, 0.1 mM EDTA and 1 mM ATP and 3 units of T4 polynucleotide kinase (product of Takara Shuzo Co.) was added. Phosphorylation reaction was carried out at 37°C for 60 minutes.

Then, 0.1 µg of the BanIII-NruI fragment (about 3.8 Kb) derived from pKYP10 and obtained above and about 0.5 pmoles of the DNA linker mentioned above were dissolved in 20 µℓ of T4 ligase buffer solution, and 2 units of T4 DNA ligase was added.  Ligation reaction was carried out at 4°C for 18 hours.

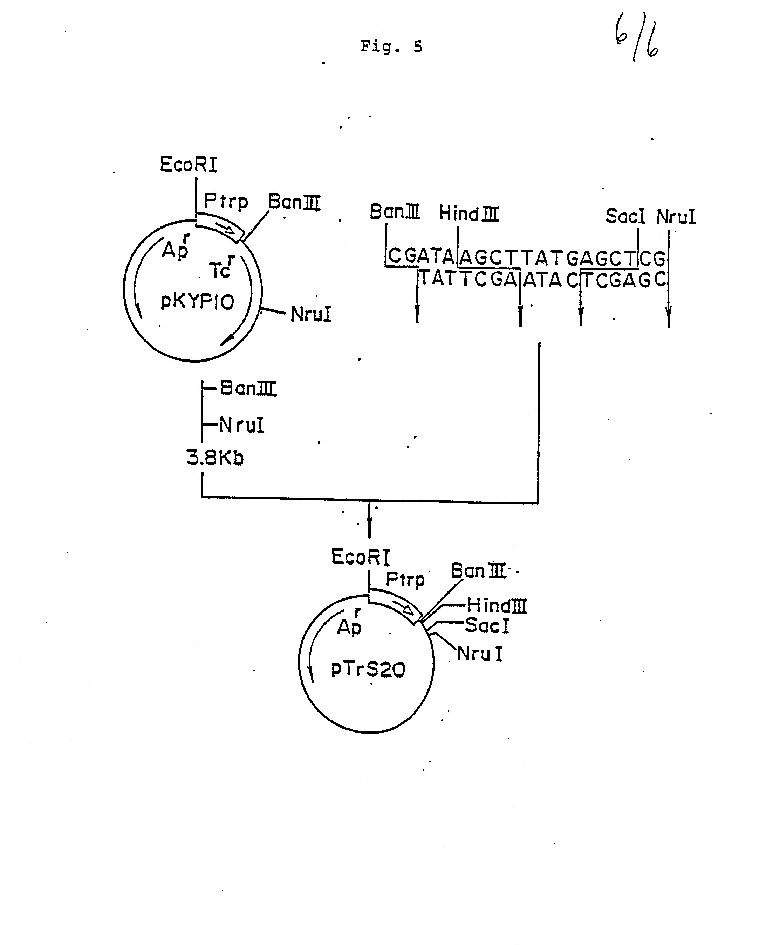

Escherichia coli HB101 [Bolivar, et al.: Gene 2, 75 (1979)] was transformed with the thus obtained mixture of the recombinant plasmids.  $Ap^R$ colonies were obtained and a plasmid DNA was recovered from the cultured cells of the colonies.  The structure of the thus obtained plasmid was confirmed by cleavage with EcoRI, BanIII, HindIII, SacI and NruI and agarose gel electrophoresis.  The plasmid was named pTrS20 (Fig. 5).  It was confirmed by the method of Sanger using M13 phage that the nucleotide sequence around the BanIII and HindIII sites of pTrS20 was as follows.

```
                    BanIII  HindIII          SacI     NruI
        SD
        sequence
        AAGG  GTAT CGATA  AGCTT  ATG  AGCT  CG  CGA
                                      Met
```

Claims

1. A fish prolactin polypeptide having the amino acid sequence as illustrated in Table 1 and derivatives thereof displaying fish prolactin polypeptide activity obtained by deletion, addition or substitution of amino acid residues in said amino acid sequence.

2. The polypeptide according to claim 1, having the amino acid sequence as illustrated in Table 2 or Table 3.

3. The polypeptide according to Claim 1, wherein the fish prolactin and the derivatives are hormones having the effect of stimulating the growth of Clupeiformes.

4. A DNA coding for a fish prolactin polypeptide or a derivative thereof.

5. The DNA according to Claim 4, wherein the fish prolactin polypeptide has the amino acid sequence as illustrated in Table 1 and the derivative has the amino acid sequence as illustrated in Table 2 or Table 3, respectively.

6. A recombinant DNA comprising a vector DNA and a DNA according to any of Claims 4 or 5.

7. The recombinant DNA according to Claim 6, which is one of the plasmids pSPRLB1, pSPRLQ28 or pSPRLQ52.

8. A microorganism containing a recombinant DNA according to any of Claims 6 or 7.

0201882

9. The microorganism according to Claim 8, wherein the microorganism belongs to <u>Escherichia</u> <u>coli</u>.

10. A process for producing a fish prolactin polypeptide or a derivative thereof, which comprises culturing in a nutrient medium a microorganism containing a recombinant DNA wherein a DNA coding for the fish prolactin polypeptide or the derivative thereof is incorporated, accumulating the fish prolactin polypeptide or the derivative thereof in the medium and recovering the polypeptide or the derivative therefrom.

# FIG.1(1)

FIG.1 (2)

Fig: 2

signal peptide    coding region

Fig. 3

Fig. 4

Fig. 5

EcoRI

Ptrp BanⅢ

Apʳ

Tcʳ

pKYPIO

NruI

BanⅢ HindⅢ          SacI NruI

CGATA|AGCTTATGAGCT|CG
TATTCGA|ATA C|TCGAGC|

─BanⅢ

─NruI

3.8Kb

EcoRI

Ptrp        BanⅢ

─HindⅢ

Apʳ        ─SacI

pTrS20        NruI